# EUROPEAN PATENT APPLICATION

(11) **EP 1 041 079 A2**
(43) Date of publication of application: **04.10.2000**
(21) Application number: 00400847.0
(22) Date of filing: 28.03.2000
(51) Int. Cl.: C07F 15/00, B01J 31/24, C07C 67/303, C07B 53/00

(54) **Ruthenium-optically active phosphine complex mixture, process for the preparation thereof and asymmetric hydrogenation catalyst comprising same**

(30) Priority: 31.03.1999 JP 9364499
(71) Applicant: Takasago International Corporation, Tokyo (JP)
(72) Inventor: Okeda, Yoshiki, Takasago International Corp., Hiratsuka-shi, Kanagawa (JP); Hashimoto, Tsutomu, Takasago International Corp., Hiratsuka-shi, Kanagawa (JP); Hori, Yoji, Takasago International Corp., Hiratsuka-shi, Kanagawa (JP); Hagiwara, Toshimitsu, Takasago International Corp., Hiratsuka-shi, Kanagawa (JP)
(74) Representative: Uchida, Kenji

(57) **Abstract**

The present invention provides a ruthenium-optically active phosphine complex mixture comprising three complexes represented by the following general formulae (I), (II) and (III), respectively:

[Ru(R¹-SEGPHOS) (sol)₄]I₂ (I)

[Ru(R¹-SEGPHOS) (sol)₄]I(anion) (II)

[RuI(R¹-SEGPHOS) (sol)₃] (anion) (III)

wherein sol represents a nitrile-based solvent; R¹-SEGPHOS represents an optically active tertiary phosphine represented by the following general formula (IV): 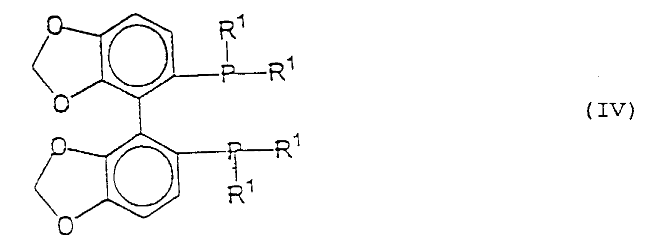 wherein R¹ represents an aryl group which may have a substituent selected from the group consisting of a C₁₋₄ lower alkyl group, a C₁₋₄ lower alkoxy group, a C₁₋₄ lower alkylamino group and a halogen atom, or a C₃₋₉ cycloalkyl group; and anion represents a perfluoroalkylsulfonyl anion, a process for the preparation thereof and an asymmetric hydrogenation catalyst comprising the ruthenium-optically active phosphine complex mixture.

## Description

### FIELD OF THE INVENTION

The present invention relates to a novel ruthenium-optically active phosphine complex mixture, a process for the preparation thereof and an asymmetric hydrogenation catalyst comprising the ruthenium-optically active phosphine complex mixture.

This mixture is useful as a catalyst for various organic synthesis reactions, particularly asymmetric hydrogenation reaction and provides an asymmetric hydrogenation catalyst particularly useful for the preparation of optically active 4-methyl-2-oxetanone, which is used as a starting material of polymer, a starting material in the synthesis of medicine or an intermediate product in the art of organic synthesis of liquid crystal material, etc.

### BACKGROUND OF THE INVENTION

Heretofore, many transition metal complexes have been used as catalysts for organic synthesis reaction. In particular, a metal complex made of metallic ruthenium and optically active tertiary phosphine is well known as a catalyst for asymmetric hydrogenation reaction. For example, a ruthenium-optically active phosphine complex comprising as a ligand an optically active tertiary phosphine such as 2,2'-bis(diphenylphosphino)-1,1'-binaphthyl (hereinafter simply referred to as "BINAP") is known in JP-A-63-135397 (The term "JP-A" as used herein means an "unexamined published Japanese patent application"), JP-A-63-145492, JP-A-5-111639, JP-A-63-145291, JP-A-10-139791, etc.

JP-A-10-182678 discloses a process for the preparation of SEGPHOS's and [RuX(arene)(SEGPHOS)] (in which SEGPHOS represents [(5,6),(5',6')-bis(methylenedioxy)biphenyl-2,2'-diyl]bis (diphenylphosphine), X represents a halogen atom, and arene represents a hydrocarbon having a benzene ring), Ru₂X₄(SEGPHOS)₂NEt₃ and Ru(methylallyl)₂ (SEGPHOS), which are ruthenium complexes having SEGPHOS as a ligand.

Further, [Ru(BINAP) (CH₃CN)₄]₂⁺X⁻(Y⁻) (in which X represents a halogen atom, and Y represents a halogen atom or BF₄), [RuX(BINAP) (CH₃CN)₃]⁺X⁻, RuX₂(BINAP) (CH₃CN)₂, etc., which are complexes analogous to the present invention, are disclosed in K. Mashima et al., *J. Chem. Soc.,* Dalton Tans., pp. 2099 - 2107 (1992).

However, these ruthenium-optically active phosphine complexes have some disadvantages in the actual industrialization. For example, even if these ruthenium-optically active phosphine complexes are used, it leaves something to be desired in the resulting catalytic activity or asymmetric yield depending on the reaction or reaction substrate in question.

On the other hand, 4-methyl-2-oxetanone (also referred to as "β-butyrolactone" or "α-methyl-β-propiolactone") has heretofore been used as a starting material of polymer. In recent years, 4-methyl-2-oxetanone has been noted useful particularly when it is in optically active compound as disclosed in JP-A-6-256482, JP-A-6-329768, JP-A-7-53694, JP-A-8-53540 and JP-A-8-127645.

As processes for the preparation of optically active 4-methyl-2-oxetanone there have been reported the following processes:
(a) A process which comprises subjecting 3-bromobutyric acid obtained by adding hydrobromic acid to crotonic acid to optical resolution with optically active naphthylethylamine, and then cyclizing the product (J. Reid Shelton et al., *Polymer Letters,* Vol. 9, pp. 173 - 178 (1971) , T. Sato et al., *Tetrahedron Lett.,* Vol. 21, pp. 3377 - 3380 (1980)).
(b) A process which comprises reacting optically active 3-hydroxybutyric acid with triethylorthoacetic acid to obtain optically active 2-ethoxy-2,6-dimethyl-1,3-dioxane-4-on, and then subjecting the product to thermal decomposition (A. Griesbeck et al., *Helv. Chim. Act*., Vol. 70, pp. 1320 - 1325 (1987), R. Breitschuh et al., *Chimia*, Vol. 44, 216 - 218 (1990)).
(c) A process which comprises reacting optically active 3-hydroxybutyric acid ester with methanesulfonyl chloride so that the hydroxyl group is mesylated, subjecting the resulting ester to hydrolysis, and then subjecting the hydrolyzate to condensation cyclization with sodium hydrogencarbonate (Y. Zhang et al., *Macromolecules,* Vol. 23, pp. 3206 - 3212 (1990)).
   As an example of processes for preparation of optically active 4-methyl-2-oxetanone using the foregoing ruthenium-optically active phosphine complexes there has been reported the following process:
(d) A process which comprises subjecting 4-methylene-2-oxetanone (also referred to as "diketene") to asymmetric hydrogenation in an aprotic solvent such as methylene chloride and tetrahydrofuran in the presence of [RuCl[(S)- or (R)-BINAP(benzene)]Cl or Ru₂Cl₄[(S)- or (R)-BINAP]₂NEt₃ (in which Et represents an ethyl group) as a catalyst (T. Ohta et al., *J. Chem. Soc.,* Chem. Commun., 1725 (1992)).

However, these processes have the following disadvantages. In other words, the process (a) requires the use of a special optically active amine as an optical resolving agent in an amount equimolecular with the starting material. Further, the process (a) involves the production of unnecessary enantiomer. Thus, this process is highly wasteful and is not economically advantageous.

Further, the processes (b) and (c) are disadvantageous in that optically active 3-hydroxybutyric acid or its ester as a starting compound cannot be easily synthesized. In other words, it is necessary that an optically active poly-3-hydroxybutyric acid ester in which microorganisms are produced be subjected to thermal decomposition or that 4-methylene-2-oxetanone be subjected to alcoholysis reaction to obtain an acetoacetic acid ester which is then subjected to asymmetric reduction. Thus, these processes require many steps and thus are complicated.

The process (d) gives solutions to many of the foregoing problems with the processes (a) to (c) but still has some problems. In other words, the catalytic activity thus given is low and the required reaction time is long. Further, the resulting product has an optical purity of as low as 70 to 92%e.e. Further, as reported in JP-A-6-128245, JP-A-7-188201, and JP-A-7-206885, this process has been improved but still leaves something to be desired on an industrial basis because the resulting catalytic activity is still low.

### SUMMARY OF THE INVENTION

An object of the present invention is to provide a novel ruthenium-optically active phosphine complex mixture particularly useful as a catalyst that exhibits a high catalytic activity and a high asymmetric yield in asymmetric reaction, i.e., allows the production of a product having a high optical purity and a process for the preparation of optically active 4-methyl-2-oxetanone or the like having a high optical purity useful as a starting material of polymer in the presence of a catalyst made of the complex mixture at a high efficiency in a short period of time.

The inventors made extensive studies of solution to the foregoing problems. As a result, it was found that a novel ruthenium-optically active phosphine complex mixture which has successfully been developed has an extremely high catalytic activity and can be widely used as an asymmetric synthesis catalyst and the use of this ruthenium-optically active complex mixture as a catalyst for asymmetric hydrogenation reaction of 4-methylene-2-oxetanone makes it possible to produce optically active 4-methyl-2-oxetanone having a high optical purity at a high efficiency in a short period of time. Thus, the present invention has been worked out.

In other words, the present invention is as follows:
(1) A ruthenium-optically active phosphine complex mixture comprising three complexes represented by the following general formulae (I), (II) and (III), respectively:

   [Ru(R¹-SEGPHOS) (sol)₄]I₂ (I)

   [Ru(R¹-SEGPHOS) (sol)₄]I(anion) (II)

   [RuI(R¹-SEGPHOS) (sol)₃] (anion) (III)

   wherein sol represents a nitrile-based solvent; R¹-SEGPHOS represents an optically active tertiary phosphine represented by the following general formula (IV): 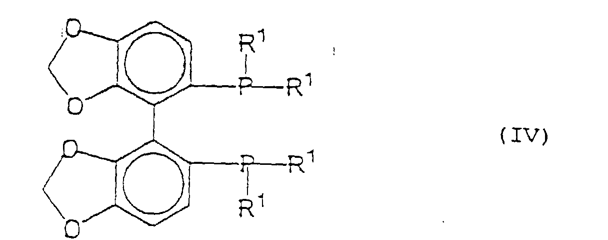 wherein R¹ represents an aryl group which may have a substituent selected from the group consisting of a C₁₋₄ lower alkyl group, a C₁₋₄ lower alkoxy group, a C₁₋₄ lower alkylamino group and a halogen atom, or a C₃₋₈ cycloalkyl group; and anion represents a perfluoroalkylsulfonyl anion.
(2) A process for the preparation of a ruthenium-optically active phosphine complex mixture described in (1) above, which comprises reacting a ruthenium-optically active phosphine complex represented by the following general formula (V):

   [RuI(arene)(R¹-SEGPHOS)]I (V)

   wherein arene represents a hydrocarbon having a benzene ring; and R¹-SEGPHOS represents an optically active tertiary phosphine represented by the following general formula (IV) : 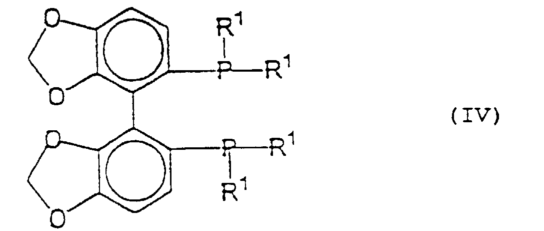 wherein R¹ is as defined in the general formula (IV) in (1) above,
   with a perfluoroalkylsulfonic acid salt represented by the following general formula (VI)

   Mₑ(anion)_{f} (VI)

   wherein M represents a mono-, di- or trication selected from the group consisting of an alkaline metal cation, an alkaline earth metal cation and an ammonium cation; anion represents a perfluoroalkylsulfonyl anion; e is 1; and f is an integer of from 1 to 3, with the proviso that when M is a monocation, f is 1, when M is a dication, f is 2, and when M is a trication, f is 3,
   in a nitrile-based solvent.
(3) A process for the preparation of a ruthenium-optically active phosphine complex mixture described in (1) above, which comprises reacting a compound represented by the following general formula (VII) :

   [RuI₂(arene)]₂ (VII)

   wherein arene represents a hydrocarbon having a benzene ring,
   an optically active phosphine represented by the following general formula (IV): 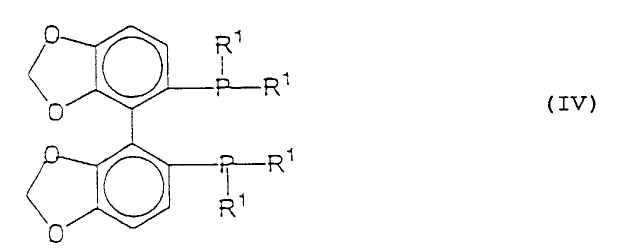 wherein R¹ is as defined in the general formula (IV) in (1) above,
   and a perfluoroalkylsulfonic acid salt represented by the following general formula (VI):

   Mₑ(anion)_{f} (VI)

   wherein M, anion, e and f are as defined in (2) above, in a nitrile-based solvent.
(4) A catalyst for asymmetric hydrogenation of 4-methylene-2-oxetanone comprising as a main component a ruthenium-optically active phosphine complex mixture described in (1) above.
(5) A catalyst for asymmetric hydrogenation of 4-methylene-2-oxetanone which is a ruthenium-optically active phosphine complex mixture obtained by the process described in (2) or (3) above.

### BRIEF DESCRIPTION OF THE DRAWINGS

Fig. 1 illustrates the structural formula of [Ru(R¹-SEGPHOS)(sol)₄]I₂, [Ru(R¹-SEGPHOS)(sol)₄]I(anion) and [RuI (R¹-SEGPHOS)(sol)₃] (anion), which constitute the ruthenium-iodo-optically active phosphine complex mixture according to the invention;
Fig. 2 is a graph illustrating ³¹P NMR spectrum of the ruthenium-iodo-optically active phosphine complex mixture according to the invention obtained in Example 1;
Fig. 3 is a graph illustrating ³¹P NMR spectrum of the ruthenium-iodo-optically active phosphine complex mixture according to the invention obtained in Example 2;
Fig. 4 is a graph illustrating ³¹P NMR spectrum of the ruthenium-iodo-optically active phosphine complex mixture according to the invention obtained in Example 3;
Fig. 5 is a graph illustrating ³¹P NMR spectrum of the ruthenium-iodo-optically active phosphine complex mixture according to the invention obtained in Example 4;
Fig. 6 is a graph illustrating ³¹P NMR spectrum of the ruthenium-iodo-optically active phosphine complex mixture according to the invention obtained in Example 5; and
Fig. 7 is a graph illustrating ³¹P NMR spectrum of the ruthenium-iodo-optically active phosphine complex mixture according to the invention obtained in Example 6.

### DETAILED DESCRIPTION OF THE INVENTION

The present invention will be further described hereinafter.

The foregoing ruthenium-iodo-optically active phosphine complexes (hereinafter collectively abbreviated as "[RuIₐ(R¹-SEGPHOS) (sol)_{b}] (I)_{c}(anion)_{d} (in which a, b, c and d represent an integer of 0, 4, 2 and 0, respectively, 0, 4, 1 and 1, respectively, or 1, 3, 0 and 1, respectively) represented by the general formulae (I) , (II) and (III) can be obtained by the two methods previously mentioned. The two methods will be described below with reference to the reaction formulae 1 and 2, respectively:

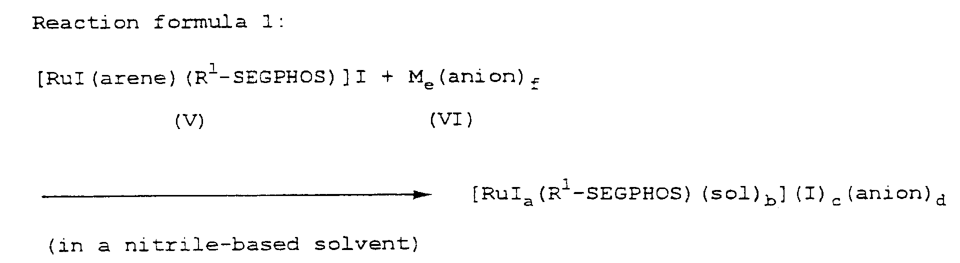

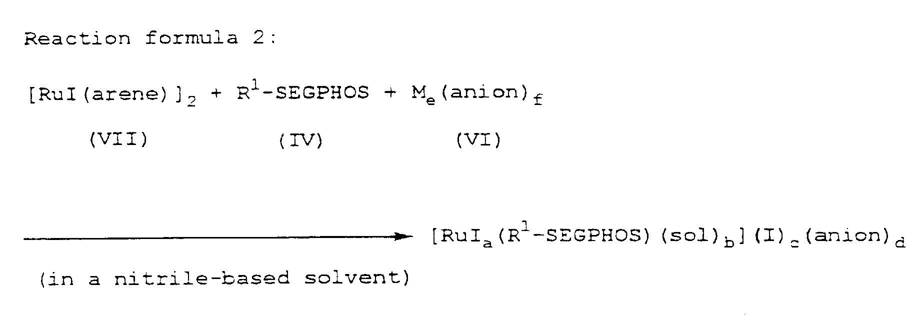

In other words, the ruthenium-iodo-optically active phosphine complex can be obtained by a process which comprises heating the foregoing ruthenium-phosphine complex (V) and perfluoroalkylsulfonic acid salt (VI) with stirring in a nitrile-based solvent in a reaction vessel in which the air within had been replaced by an inert gas such as nitrogen to effect reaction as shown in Reaction Formula 1, distilling off the nitrile-based solvent, and then stirring the residue in a two-phase solvent consisting of methylene chloride and water at room temperature so that it is cleaned. The ruthenium-iodo-optically active phosphine complex can be also obtained by a process which comprises heating the foregoing ruthenium complex (VII), phosphine ligand (IV) and perfluoroalkylsulfonic acid salt (VI) in a nitrile-based solvent with stirring to effect reaction as shown in Reaction Formula 2, distilling off the nitrile-based solvent, and then stirring the residue in a two-phase solvent consisting of methylene chloride and water at room temperature so that it is cleaned.

The complex thus obtained is a mixture of [Ru(SEGPHOS) (sol)₄]²⁺(anion)₂, [Ru(SEGPHOS) (sol)₄]²⁺(I⁻), [Ru(SEGPHOS)(sol)₄]²⁺I⁻(anion) and [RuI(SEGPHOS) (sol)₃]⁺ (anion) as judged by the results of ³¹P NMR and K. Mashima et al., *J. Chem. Soc.,* Dalton Trans., pp. 2099 - 2107 (1992).

The perfluoroalkylsulfonic acid salt represented by the general formula (VI) to be used in the invention is used to replace iodine ions present in the catalyst precursor represented by the general formula (V) or (VII) partly or entirely by perfluoroalkylsulfon anions. The formation of a salt by metal cations or ammonium cations in the perfluoroalkylsulfonic acid salt represented by the general formula (VI) and iodine anions causes iodine ions to be excluded from the system. Accordingly, the metal cations or ammonium cations in the perfluoroalkylsulfonic acid salt are not specifically limited so far as they form a salt with iodine anions. The complex compositions produced by the foregoing two preparation processes have no big difference.

Referring to the ruthenium-optically active phosphine complex (V) as a starting material of [RuIₐ(R¹-SEGPHOS) (sol)_{b}] (I)_{c}(anion)_{d} according to the present invention, examples of the aryl group represented by R¹ in R¹-SEGPHOS (IV) include phenyl group, 2-naphthyl group, substituted phenyl group such as p-substituted phenyl group, m-substituted phenyl group and m-disubstituted phenyl group, and substituted 2-naphthyl group such as 6-substituted-2-naphthyl group. Examples of substituents which may substitute on phenyl group and naphthyl group include lower (The term "lower" as used herein is meant to indicate C₁₋₄ straight-chain or branched chain) alkyl group such as methyl group, ethyl group, n-propyl group, iso-propyl group, n-butyl group, sec-butyl group and tert-butyl group, lower alkoxy group, lower alkylamino group, and halogen atom such as chlorine. Particularly preferred examples of C₃₋₈ cycloalkyl group represented by R¹ are cyclopentyl group and cyclohexyl group.

R¹-SEGPHOS can be obtained from 3,4-methylenedioxybenzene by the following five steps in the method described in JP-A-10-182678. In some detail, diphenyl(3,4-methylenedioxyphenyl)phosphine oxide is prepared from a Grignard reagent of 3,4-methylenedioxybromobenzene and diphenylphosphinyl chloride. Diphenyl (3,4 -methylene dioxyphenyl)phosphine oxide is then subjected to iodation. The material thus iodated is then allowed to undergo coupling reaction in the presence of copper powder to obtain R¹-SEGPHOS oxide in racemic form. The product is subjected to optical resolution, and then subjected to reduction with trichlorosilane to obtain optically active R¹-SEGPHOS.

Specific examples of the tertiary phosphine will be listed below. All these tertiary phosphines exist in (R) isomer and (S) isomer, but their description is omitted hereinafter. [(5,6), (5',6')-bis(methylenedioxy)biphenyl-2,2'-diyl]bis (diphenylphosphine) (hereinafter simply referred to as "SEGPHOS")
[(5,6),(5',6')-bis(methylenedioxy)biphenyl-2,2'-diyl]bis (di-p-tolyl-phosphine) (hereinafter referred to as "T-SEGPHOS")
[(5,6), (5',6')-bis (methylenedioxy)biphenyl-2,2'-diyl] bis[di-(p-tert-butylphenyl)phosphine] (hereinafter simply referred to as "tBu-SEGPHOS")
[(5,6), (5',6')-bis (methylenedioxy)biphenyl-2,2'-diyl] bis(di-m-tolyl-phosphine) (hereinafter simply referred to as "m-T-SEGPHOS")
[(5,6), (5',6')-bis(methylenedioxy)biphenyl-2,2'-diyl]bis [di- (3,5-dimethylphenyl)phosphine] (hereinafter simply referred to as "DM-SEGPHOS")
[(5,6), (5',6')-bis (methylenedioxy)biphenyl-2,2' -diyl]bis [di-(3,5-di-tert-butylphenyl)phosphine] (hereinafter simply referred to as "DtBu-SEGPHOS")
[(5,6), (5',6')-bis (methylenedioxy)biphenyl-2,2'-diyl]bis [di-(p-methoxyphenyl)phosphine] (hereinafter simply referred to as "MeO-SEGPHOS")
[(5,6),(5',6') -bis(methylenedioxy)biphenyl-2,2'-diyl]bis [di-(p-chlorophenyl)phosphine] (hereinafter simply referred to as "p-Cl-SEGPHOS")
[(5,6),(5',6') -bis(methylenedioxy)biphenyl-2,2'-diyl]bis (di-2-naphthylphosphine) (hereinafter simply referred to as "Naph-SEGPHOS")
[(5,6),(5',6')-bis(methylenedioxy)biphenyl-2,2'-diyl]bis (dicyclopentylphosphine) (hereinafter simply referred to as "cpSEGPHOS")
[(5,6),(5' ,6')-bis(methylenedioxy)biphenyl-2,2'-diyl]bis (dicyclohexylphosphine) (hereinafter simply referred to as "CySEGPHOS")

In the present invention, the ruthenium-optically active phosphine complex represented by [RuI (arene) (R¹-SEGPHOS)]I can be obtained by the method described in JP-A-2-191289 and JP-A-5-111639. Briefly discussing, the ruthenium-optically active phosphine complex can be obtained by heating the complex represented by the general formula (VII) and R¹-SEGPHOS represented by the general formula (IV) to a temperature of 50°C with stirring for about 2 hours.

Examples of the hydrocarbon having benzene ring represented by arene in the general formulae (V) and (VII) include benzene, toluene, xylene, mesitylene, p-cymene, hexamethylbenzene, methoxybenzene, and benzoic acid methyl.

A specific example of the ruthenium-optically active phosphine complex represented by the general formula (V) is the following compound:

[RuI (arene) (R¹-SEGPHOS)]I (V)

wherein arene represents benzene or p-cymene; and R¹-SEGPHOS represents SEGPHOS, T-SEGPHOS, tBu-SEGPHOS, m-T-SEGPHOS, DM-SEGPHOS, DtBu-SEGPHOS, MeO-SEGPHOS, p-Cl-SEGPHOS, Naph-SEGPHOS, cpSEGPHOS or CySEGPHOS as mentioned above.

The ruthenium-iodo complex represented by the general formula (VII) can be obtained by the method described in Zelonka (R.A.Zelonka et *a*., *Can. J. Chem.*, Vol. 50, 3063 (1972)). Specific examples of the ruthenium-optically active phosphine complex thus obtained include the following compounds:
[RuI₂(benzene)]₂
[RuI₂(p-cymene)]₂

On the other hand, examples of the salt represented by the general formula (VI) as a starting material to be used in the preparation of [RuIₐ(R¹-SEGPHOS) (sol)_{b}] (I)_{c}(anion)_{d} from the ruthenium complexes represented by the general formulae (V) and (VII) include the following compounds (a) to (d):
(a) Lithium trifluoromethanesulfonate (lithium triflate) (hereinafter simply referred to as "LiOTf"), sodium trifluoromethanesulfonate (sodium triflate) (hereinafter simply referred to as "NaOTf"), potassium trifluoromethanesulfonate (potassium triflate) (hereinafter simply referred to as "KOTf"), Mg(OTf)₂, Al(OTf)₃, AgOTf, NH₄OTf;
(b) Lithium nonafluorobutanesulfonate (hereinafter simply referred to as "LiONf"), sodium nonafluorobutanesulfonate (hereinafter simply referred to as "NaONf") , potassium nonafluorobutanesulfonate (hereinafter simply referred to as "KONf") ;
(c) Lithium heptadecafluorooctanesulfonate (hereinafter simply referred to as "LiOhepDf"), sodium heptadecafluorooctanesulfonate (hereinafter simply referred to as "NaOhepDf"), potassium heptadecafluorooctanesulfonate (hereinafter simply referred to as "KOhepDf");
(d) Sodium undecafluorocyclohexanesulfonate (hereinafter simply referred to as "NaOuDCyf"), potassium undecafluorocyclohexanesulfonate (hereinafter simply referred to as "KOuDCyf")

The amount of the salt to be used is preferably from about 0.25 to 2 mols, particularly preferably from about 0.5 to 1 mol per mol of ruthenium atom in the ruthenium complexes (V) and (VII).

Examples of the nitrile-based solvent to be used in Reaction Formula 1 and Reaction Formula 2 include acetonitrile, benzonitrile, and mixture thereof. Preferred nitrile-based solvents are acetonitrile and benzonitrile, and acetonitrile is particularly preferred.

The temperature at which [RuIₐ(R¹-SEGPHOS)(sol)_{b}](I)_{c}(anion)_{d} of the present invention is prepared according to Reaction Formula 1 and Reaction Formula 2 is from about 60°C to 90°C, preferably from 70°C to 80°C. The reaction time is preferably from about 10 to 40 hours, particularly preferably from about 15 to 20 hours. After the termination of the reaction, the resulting hydrophobic organic solvent layer is then withdrawn. The solvent is then distilled off. The residue can be then purified by drying or the like to obtain the desired complex mixture [RuIₐ(R¹-SEGPHOS) (sol)_{b}] (I)_{c}(anion)_{d} (in which a, b, c and d represent an integer of 0, 4, 2 and 0, respectively, 0, 4, 1 and 1, respectively, or 1, 3, 0 and 1, respectively) of the invention.

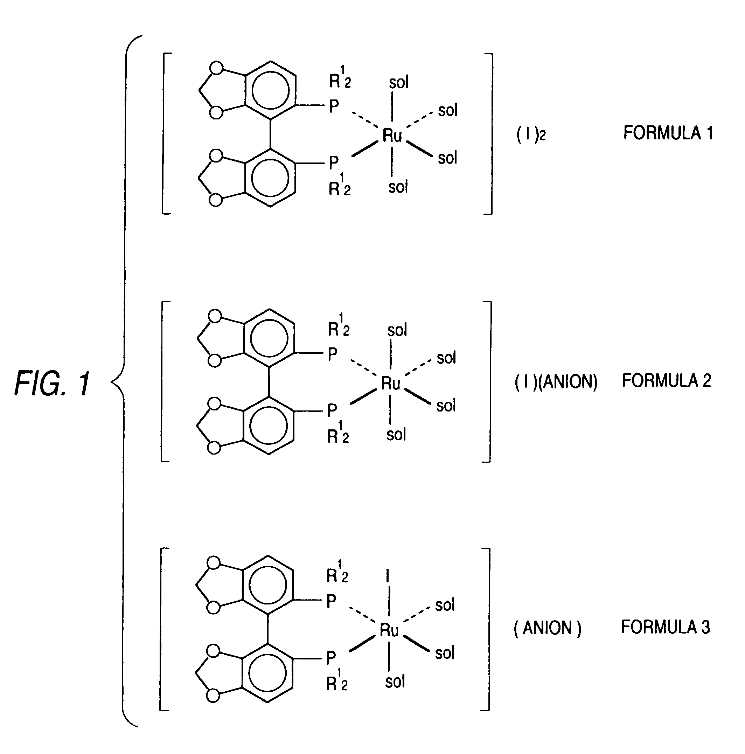

[RuIₐ(R¹-SEGPHOS)(sol)_{b}](I)_{c}(anion)_{d} thus obtained is a complex comprising a nitrile-based solvent incorporated therein as a ligand obtained by the elimination of arene molecule from the compounds (V) and (VII). It is also a mixture of complexes having iodine atoms partly or entirely replaced by other perfluoroalkylsulfonyl anions. In other words, specific examples of the complex mixture thus obtained, if represented by the general formula comprising ruthenium, iodine, perfluoroalkylsulfonyl anion and optically active tertiary phosphine, include those having structures corresponding to the general formulae 1 to 3 in Fig. 1: [Ru(R¹-SEGPHOS) (sol)₄]I₂, [Ru(R¹-SEGPHOS) (sol)₄]I(anion) and [RuI(R¹-SEGPHOS)(sol)₃] (anion) and [Ru(R¹-SEGPHOS) (sol)₄] (anion)₂. These complexes can be obtained in either (R) isomer or (S) isomer depending on the absolute configuration of R¹-SEGPHOS (IV) used. However, their description is omitted.

In the foregoing general formulae, R¹-SEGPHOS is SEGPHOS, T-SEGPHOS, tBu-SEGPHOS, m-T-SEGPHOS, DM-SEGPHOS, DtBu-SEGPHOS, MeO-SEGPHOS, p-Cl-SEGPHOS, Naph-SEGPHOS, cpSEGPHOS or CySEGPHOS as mentioned above, sol is acetonitrile (CH₃CN) or benzonitrile (PhCN), and anion is OTf, ONf, OhepDf or OuDCyf as mentioned above. [Ru(R¹-SEGPHOS) (sol)₄] (anion)₂ is not an iodine complex and has a low activity and thus needs to be produced in a small amount. Even if this complex is contained in the complex mixture, there causes no special problem. Thus, the iodine complex mixture of the present invention can be used as a mixture in the reaction without being subjected to separation and purification.

The mixture of the present invention has an extremely high catalytic activity and thus can be widely used as an asymmetric synthesis catalyst. In particular, the use of this mixture as a catalyst for the asymmetric hydrogenation reaction of 4-methylene-2-oxetanone makes it possible to produce optically active 4-methyl-2-oxetanone having a high optical purity at a high efficiency in a short period of time.

The production of optically active 4-methyl-2-oxetanone using [RuIₐ(R¹-SEGPHOS) (sol)_{b}] (I)_{c}(anion)_{d} may be accomplished, e.g., by the asymmetric hydrogenation of 4-methylene-2-oxetanone as a starting material and a solvent in the presence of the foregoing [RuIₐ(R¹-SEGPHOS)(sol)_{b}](I)_{c}(anion)_{d} in a nitrogen atmosphere at a hydrogen pressure of from 0.5 to 15 MPa and a temperature of from room temperature to 100°C in a pressure vessel. 4-Methylene-2-oxetanone to be used as a starting material in this process can be easily synthesized by the thermal decomposition of acetic acid or acetic anhydride, e.g., according to the method reported by R. J. Clemens et al., *Chem. Rev.*, Vol. 86, pp. 241 - 318 (1986).

[RuIₐ(R¹-SEGPHOS) (sol)_{b}] (I)_{c}(anion)_{d} as an asymmetric hydrogenation catalyst can be selectively in either (R) isomer or (S) isomer to obtain 4-methyl-2-oxetanone having a desired absolute configuration.

In order to favorably produce optically active 4-methyl-2-oxetanone, it is preferred that [RuIₐ(R¹-SEGPHOS)(sol)_{b}](I)_{c}(anion)_{d} prepared from ruthenium-arene complex and optically active phosphine at a molar ratio of from 1 : 1.05 to 1 : 0.95 be used. In general, in order to prepare an optically active complex represented by the general formula (5), an optically active phosphine ligand is used in an amount of from about 1.05 to 1.2 equivalents per equivalent of ruthenium. By reducing the amount of the optically active phosphine ligand to 1.05 equivalents or less, the polymerization reaction of 4-methylene-2-oxetanone as a side reaction can be inhibited. When the amount of optically active phosphine falls below 0.95 equivalent, the amount of ruthenium-arene complex is excessive, giving poor economy.

The amount of [RuIₐ(R¹-SEGPHOS)(sol)_{b}](I)_{c}(anion)_{d} to be used as a catalyst is preferably from about 1/30,000 to 1/2,000 equivalent, particularly from about 1/20,000 to 1/10,000 equivalent per equivalent of 4-methylene-2-oxetanone as a starting material. When the amount of the catalyst to be used falls below 1/30,000 equivalent, the catalyst used cannot sufficiently exert its effect. On the contrary, when the amount of the catalyst to be used exceeds 1/2,000 equivalent, it gives poor economy.

The solvent to be used herein is not specifically limited so far as it is used for ordinary asymmetric hydrogenation. Specific examples of the solvent employable herein include straight-chain or cyclic ethers such as diethyl ether, tetrahydrofuran and dioxane, organic halogenated compounds such as methylene chloride, methylene bromide and dichloroethane, ketones such as acetone, methyl ethyl ketone and methyl butyl ketone, carboxylic acids such as acetic acid and propionic acid, esters such as ethyl acetate, butyl acetate and methyl 3-hydroxybutyrate, aromatic compounds such as toluene and benzene, alcohols such as methanol, ethanol, isopropanol, tert-butyl alcohol and 1,3-butanediol, and mixture thereof. In order to raise the rate of asymmetric hydrogenation, the foregoing solvent may comprise water incorporated therein in an amount of about 1%.

The asymmetric hydrogenation reaction temperature and time depend on the kind of the catalyst used and other reaction conditions. In practice, however, the asymmetric hydrogenation reaction may be effected at a temperature of from room temperature to 100°C, particularly preferably from about 30°C to 70°C for about 0.5 to 40 hours. The hydrogen pressure to be applied is preferably from about 5 to 150 kg/cm², particularly preferably from about 20 to 100 kg/cm². After the termination of the reaction, the reaction product can be purified, e.g., by removal of solvent and other ingredients by distillation to obtain a desired optically active 4-methyl-2-oxetanone having a high optical purity at a high efficiency in a short period of time.

The present invention will be further described in the following examples and comparative examples, but the present invention should not be construed as being limited thereto. For the measurement of the physical properties of the compounds obtained in the following examples, the following instruments were used:
³¹P NMR spectrum: Type DRX500 apparatus (produced by Bruker Inc.)
External standard substance: 85% Phosphoric acid
Solvent: Heavy chloroform

### (Measurement of optical purity)

Gas chromatograph: HEWLETT PACKARD 5890 SERIES II
Optically active column: Charldex G-TA 30m (produced by ASTEC Corp.)

### EXAMPLE 1

Preparation of a mixture of complexes [Ru((S)-SEGPHOS) (CH₃CN)₄] (ONf)₂, [Ru((S)-SEGPHOS) (CH₃CN)₄] (I)₂, [Ru((S)-SEGPHOS) (CH₃CN)₄] (I) (ONf) and [RuI((S)-SEGPHOS) (CH₃CN)₃] (ONf) :

The air in a 100 ml eggplant type flask was replaced by nitrogen. Into the eggplant type flask were then charged 685 mg (0.700 mmol) of [RuI₂(p-cymene)]₂, 863 mg (1.414 mmol) of (S)-SEGPHOS, 239 mg (0.707 mmol) of KONf and 35 ml of deaerated acetonitrile. The mixture was then stirred at a temperature of 80°C for 16 hours. Thereafter, the mixture was cooled to a temperature of 40°C. Acetonitrile was then recovered under reduced pressure. In a nitrogen atmosphere, the residue was then stirred with 20 ml of methylene chloride and 20 ml of deaerated water for 10 minutes. The resulting methylene chloride layer was withdrawn through a syringe, and then washed with 20 ml of deaerated water. Methylene chloride was then distilled off under reduced pressure. The resulting complex was dried at a temperature of 30°C for 4 hours. As a result, the desired compound was obtained in an amount of 1.6 g (yield: 89.7%).

³¹P NMR: δ 43.1, 45.9, 47.5 ppm.

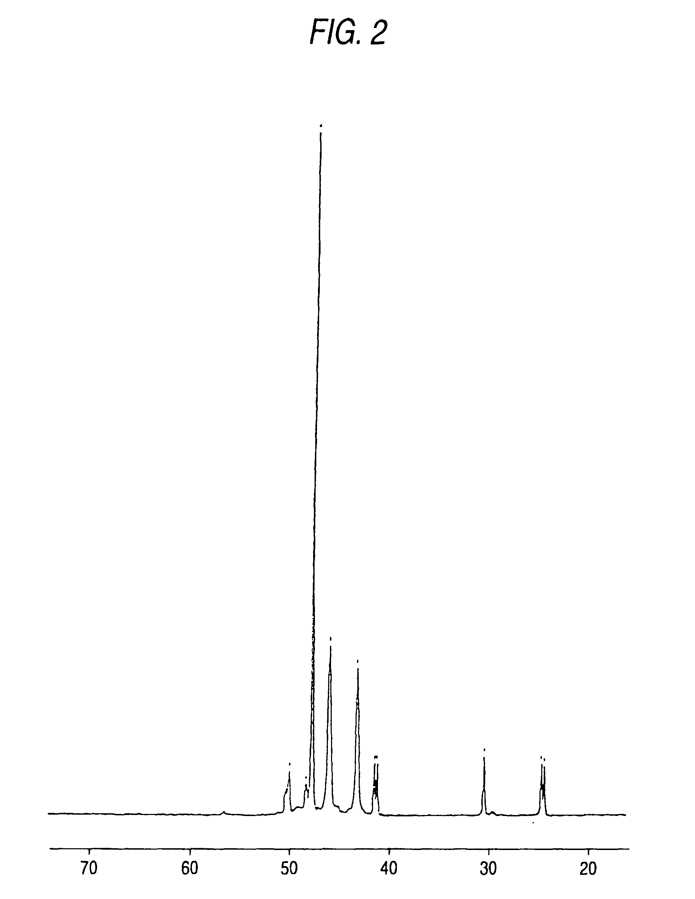

As can be seen in Fig. 2, the mixing ratio of [RuI((S)-SEGPHOS) (CH₃CN)₃] (ONf) (43.1, 45.8 ppm) to the sum (47.5 ppm) of [Ru((S)-SEGPHOS) (CH₃CN)₄](ONf)₂, [Ru((S)-SEGPHOS) (CH₃CN)₄] (I)₂ and [Ru((S)-SEGPHOS) (CH₃CN)₄] (I) (ONf) was 29 : 71.

### EXAMPLE 2

Preparation of a mixture of complexes [Ru((S)-SEGPHOS) (CH₃CN)₄] (ONf)₂, [Ru((S)-SEGPHOS) (CH₃CN)₄] (I)₂, [Ru((S)-SEGPHOS) (CH₃CN)₄] (I) (ONf) and [RuI((S)-SEGPHOS) (CH₃CN)₃](ONf) :

The air in a 20 ml eggplant type flask was replaced by nitrogen. Into the eggplant type flask were then charged 135 mg (0.122 mmol) of [RuI (p-cymene) {(S)-SEGPHOS)]I, 20.9 mg (0.0619 mmol) of KONf and 3 ml of deaerated acetonitrile. The mixture was then stirred for 16 hours. Thereafter, the mixture was cooled to a temperature of 40°C. Acetonitrile was then recovered under reduced pressure. In a nitrogen atmosphere, the residue was then stirred with 10 ml of methylene chloride and 10 ml of deaerated water for 10 minutes. The resulting methylene chloride layer was withdrawn through a syringe, and then washed with 20 ml of deaerated water. Methylene chloride was then distilled off under reduced pressure. The resulting complex was dried at a temperature of 30°C for 4 hours. As a result, the desired compound was obtained in an amount of 130 mg (yield: 84.1%).

³¹P NMR: δ 43.1, 45.8, 47.5 ppm.

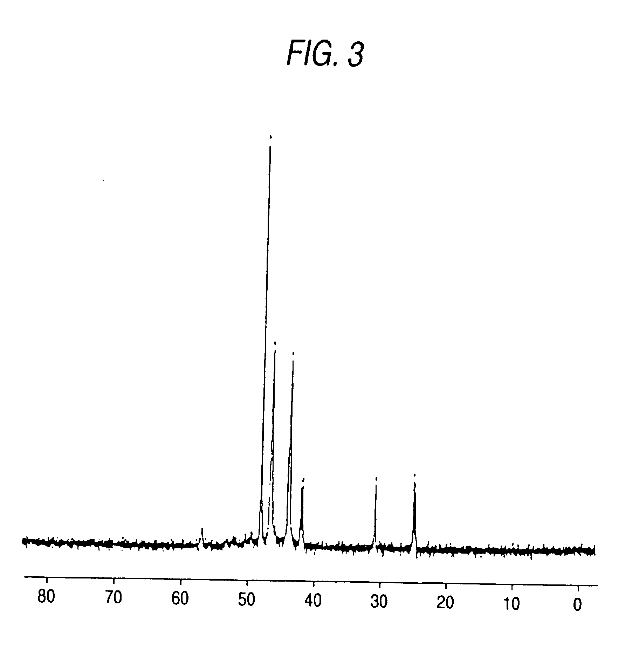

As can be seen in Fig. 3, the mixing ratio of [RuI((S)-SEGPHOS) (CH₃CN)₃](ONf) (43.1, 45.8 ppm) to the sum (47.5 ppm) of [Ru((S)-SEGPHOS) (CH₃CN)₄] (ONf)₂, [Ru((S)-SEGPHOS) (CH₃CN)₄] (I)₂ and [Ru((S)-SEGPHOS) (CH₃CN)₄] (I) (ONf) was 45 : 54.

### EXAMPLE 3

Preparation of a mixture of complexes [Ru((S)-SEGPHOS) (CH₃CN)₄] (OTf)₂, [Ru((S)-SEGPHOS) (CH₃CN)₄] (I)₂, [Ru((S)-SEGPHOS) (CH₃CN)₄] (I) (OTf) and [RuI((S)-SEGPHOS) (CH₃CN)₃](OTf) :

The air in a 20 ml eggplant type flask was replaced by nitrogen. Into the eggplant type flask were then charged 60 mg (0.0613 mmol) of [RuI₂(p-cymene)]₂,75.7 mg (0.124 mmol) of (S)-SEGPHOS, 11.7 mg (0.0619 mmol) of KOTf and 3 ml of deaerated acetonitrile. The mixture was then stirred at a temperature of 80°C for 16 hours. Thereafter, the mixture was cooled to a temperature of 40°C. Acetonitrile was then recovered under reduced pressure. In a nitrogen atmosphere, the residue was then stirred with 10 ml of methylene chloride and 15 ml of deaerated water for 10 minutes. The resulting methylene chloride layer was withdrawn through a syringe, and then washed with 15 ml of deaerated water. Methylene chloride was then distilled off under reduced pressure. The resulting complex was dried at a temperature of 30°C for 4 hours. As a result, the desired compound was obtained in an amount of 120 mg (yield: 88.1%).

³¹P NMR: δ 43.1, 45.8, 47.5 ppm.

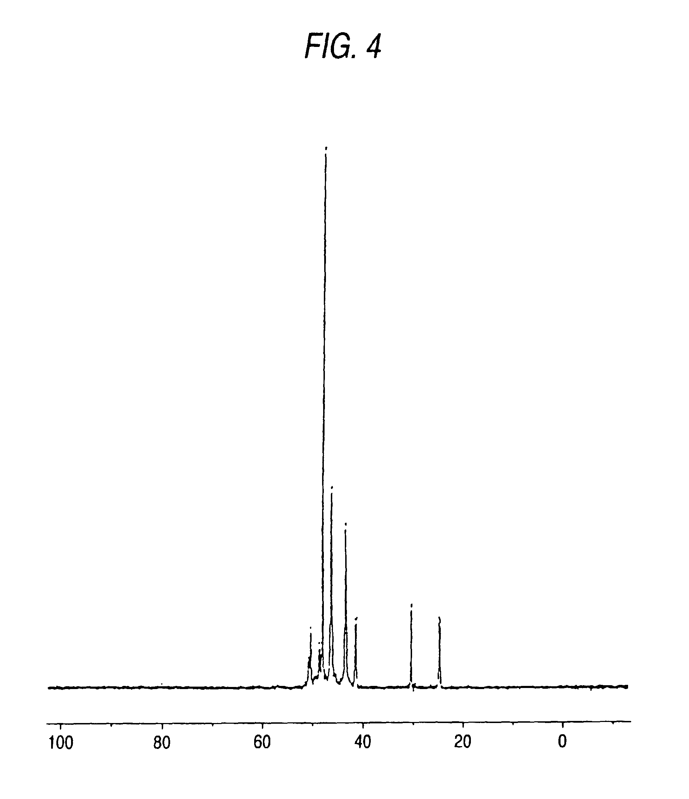

As can be seen in Fig. 4, the mixing ratio of [RuI ((S)-SEGPHOS) (CH₃CN)₃] (OTf) (43.1, 45.8 ppm) to the sum (47.5 ppm) of [Ru((S)-SEGPHOS) (CH₃CN)₄] (OTf)₂, [Ru((S)-SEGPHOS) (CH₃CN)₄] (I)₂ and [Ru((S)-SEGPHOS) (CH₃CN)₄] (I) (OTf) was 37 : 63.

### EXAMPLE 4

Preparation of a mixture of complexes [Ru((S)-SEGPHOS) (CH₃CN)₄] (OhepDf)₂, [Ru((S)-SEGPHOS) (CH₃CN)₄] (I)₂, [Ru((S)-SEGPHOS) (CH₃CN)₄] (I) (OhepDf) and [RuI((S)-SEGPHOS) (CH₃CN)₃] (OhepDf) :

The air in a 20 ml eggplant type flask was replaced by nitrogen. Into the eggplant type flask were then charged 60 mg (0.0613 mmol) of [RuI₂(p-cymene)]₂,75.7 mg (0.124 mmol) of (S)-SEGPHOS, 33.3 mg (0.0619 mmol) of KOhepDf and 3 ml of deaerated acetonitrile. The mixture was then stirred at a temperature of 80°C for 16 hours. Thereafter, the mixture was cooled to a temperature of 40°C. Acetonitrile was then recovered under reduced pressure. In a nitrogen atmosphere, the residue was then stirred with 10 ml of methylene chloride and 15 ml of deaerated water for 10 minutes. The resulting methylene chloride layer was withdrawn through a syringe, and then washed with 15 ml of deaerated water. Methylene chloride was then distilled off under reduced pressure. The resulting complex was dried at a temperature of 30°C for 4 hours. As a result, the desired compound was obtained in an amount of 150 mg (yield: 83.8%)

³¹P NMR: δ 43.2, 45.8, 47.5 ppm.

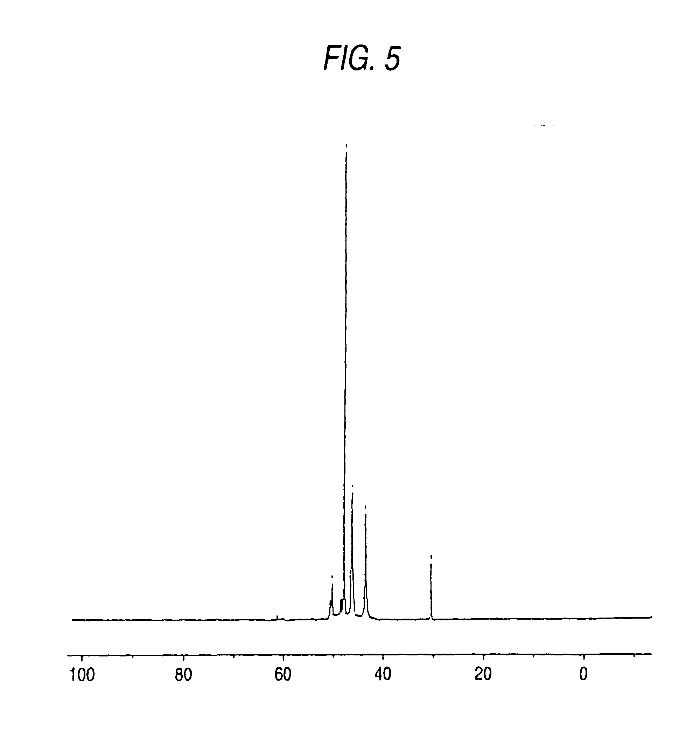

As can be seen in Fig. 5, the mixing ratio of [RuI((S)-SEGPHOS) (CH₃CN)₃] (OhepDf) (43.2, 45.8 ppm) to the sum (47.5 ppm) of [Ru((S)-SEGPHOS) (CH₃CN)₄] (OhepDf)₂, [Ru((S)-SEGPHOS) (CH₃CN)₄] (I)₂ and [Ru((S)-SEGPHOS) (CH₃CN)₄] (I) (OhepDf) was 42 : 58.

### EXAMPLE 5

Preparation of a mixture of complexes [Ru((S)-SEGPHOS) (PhCN)₄] (ONf)₂, [Ru((S)-SEGPHOS) (PhCN)₄] (I)₂, [Ru((S)-SEGPHOS) (PhCN)₄] (I) (ONf) and [RuI((S)-SEGPHOS) (PhCN)₃] (ONf) :

The air in a 20 ml eggplant type flask was replaced by nitrogen. Into the eggplant type flask were then charged 60 mg (0.0613 mmol) of [RuI₂(p-cymene)]₂,75.7 mg (0.124 mmol) of (S)-SEGPHOS, 20.9 mg (0.0619 mmol) of KONf and 3 ml of deaerated benzonitrile. The mixture was then stirred at a temperature of 80°C for 16 hours. Thereafter, the mixture was cooled to a temperature of 40°C. Benzonitrile was then recovered under reduced pressure. In a nitrogen atmosphere, the residue was then stirred with 10 ml of methylene chloride and 15 ml of deaerated water for 10 minutes. The resulting methylene chloride layer was withdrawn through a syringe, and then washed with 15 ml of deaerated water. Methylene chloride was then distilled off under reduced pressure. The resulting complex was dried at a temperature of 30°C for 4 hours. As a result, the desired compound was obtained in an amount of 140 mg (yield: 78.9%) .

³¹P NMR: δ 42.1, 44.0, 46.6 ppm.

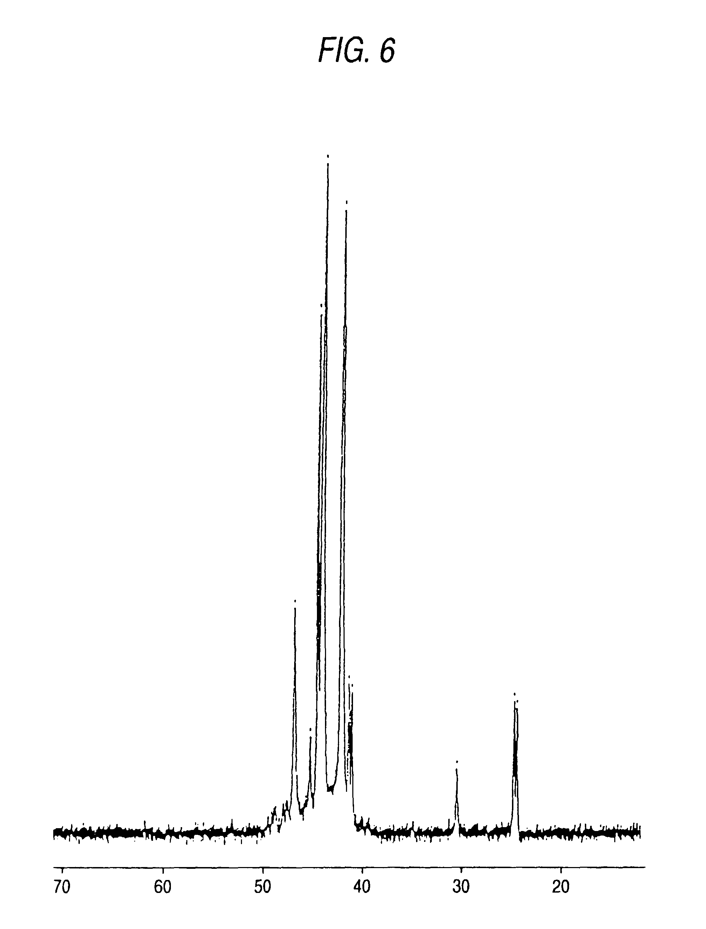

As can be seen in Fig. 6, the mixing ratio of [RuI((S)-SEGPHOS) (PhCN)₃] (ONf) (42.1, 44.0 ppm) to the sum (46.6 ppm) of [Ru((S)-SEGPHOS) (PhCN)₄] (ONf)₂, [Ru((S)-SEGPHOS) (PhCN)₄] (I)₂ and [Ru((S)-SEGPHOS) (PhCN)₄] (I) (ONf) was 84 : 16.

### EXAMPLE 6

Preparation of a mixture of complexes [Ru((S)-DM-SEGPHOS)(CH₃CN)₄] (OhepDf)₂, [Ru((S)-DM-SEGPHOS) (CH₃CN)₄](I)₂, [Ru((S)-DM-SEGPHOS) (CH₃CN)₄] (I) (OhepDf) and [RuI((S)-DM-SEGPHOS)(CH₃CN)₃] (OhepDf) :

The air in a 100 ml eggplant type flask was replaced by nitrogen. Into the eggplant type flask were then charged 166.0 mg (0.170 mmol) of [RuI₂(p-cymene)]₂,250.0 mg (0.344 mmol) of (S)-DM-SEGPHOS, 93.3 mg (0.172 mmol) of KOhepDf and 3 ml of deaerated acetonitrile. The mixture was then stirred at a temperature of 80°C for 16 hours. Thereafter, the mixture was cooled to a temperature of 40°C. Acetonitrile was then recovered under reduced pressure. In a nitrogen atmosphere, the residue was then stirred with 10 ml of methylene chloride and 15 ml of deaerated water for 10 minutes. The resulting methylene chloride layer was withdrawn through a syringe, and then washed with 15 ml of deaerated water. Methylene chloride was then distilled off under reduced pressure. The resulting complex was dried at a temperature of 30°C for 4 hours. As a result, the desired compound was obtained in an amount of 400 mg (yield: 74.6%).

³¹P NMR: δ 45.8, 44.4, 41.9 ppm.

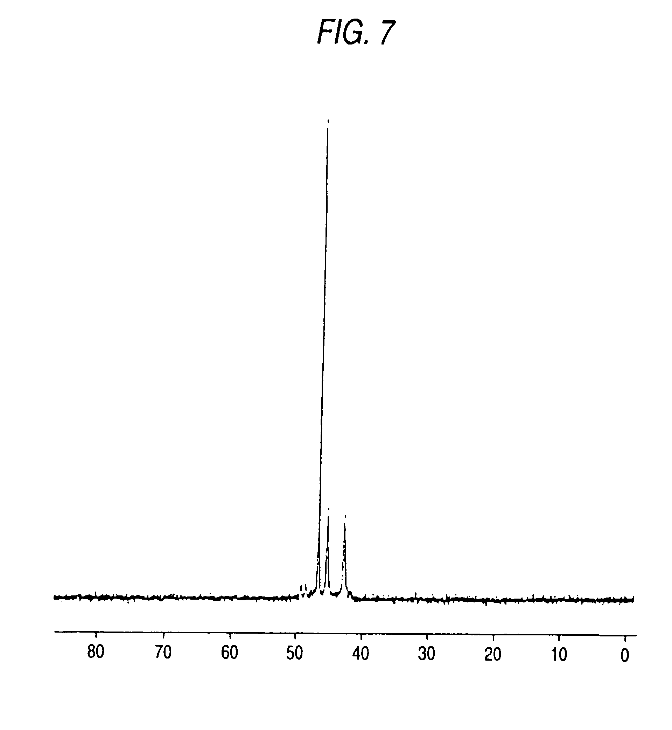

As can be seen in Fig. 7, the mixing ratio of [RuI((S) -DM-SEGPHOS) (CH₃CN)₃] (OhepDf) (44 .3, 41.9 ppm) to the sum (45.8 ppm) of [Ru((S)-DM-SEGPHOS)(CH₃CN)₄] (OhepDf)₂, [Ru((S)-DM-SEGPHOS) (CH₃CN)₄] (I)₂ and [Ru((S)-DM-SEGPHOS) (CH₃CN)₄](I)(OhepDf) was 49 : 51.

### EXAMPLE 7

### Preparation of (R)-4-methyl-2-oxetanone:

Into a 500 ml stainless steel autoclave were charged 16.7 mg (0.0132 mmol) of the mixture catalyst obtained in Example 1, 20.02 g (238.1 mmol) of 4-methylene-2-oxetanone, 80 ml of acetone and 0.45 ml of deaerated water in a nitrogen atmosphere. The mixture was then stirred at a hydrogen pressure of 4 MPa and a reaction temperature of 60°C for 16 hours. The resulting reaction solution was then subjected to distillation by a Claisen distillation apparatus to obtain a fraction having a boiling point of from 71 to 73°C/3866 Pa in an amount of 18.6 g (yield: 93.0%). The percent conversion in this reaction was 93.0%, and the catalytic activity was 16,771 as calculated in terms of number of turnovers.

As a result of comparative analysis with respect to standard product by gas chromatography, the reaction product was identified as 4-methyl-2-oxetanone. Gas chromatography (GC) using a Type Chiraldex G-TA 30m optically active column produced by ASTEC Corp. showed that the reaction product has an absolute configuration of (R) and an optical purity of 91.6%e.e,

### EXAMPLE 8

### Preparation of (R)-4-methyl-2-oxetanone:

The reaction procedure of Example 6 was followed except that 21.1 mg (0.0190 mmol) of the complex mixture catalyst obtained in Example 3 and 20.72 g (246.5 mmol) of 4-methylene-2-oxetanone were used. As a result, the desired compound was obtained in an amount of 18.5 g (yield: 89.2%). The percent conversion in this reaction was 89.2%, and the catalytic activity was 11,594 as calculated in terms of number of turnovers. The reaction product had an absolute configuration of (R) and an optical purity of 91.9%e.e.

### EXAMPLE 9

### Preparation of (R)-4-methyl-2-oxetanone:

The reaction procedure of Example 6 was followed except that 17.4 mg (0.0119 mmol) of the complex mixture catalyst obtained in Example 4 and 21.01 g (249.9 mmol) of 4-methylene-2-oxetanone were used. As a result, the desired compound was obtained in an amount of 18.1 g (yield: 86.2%). The percent conversion in this reaction was 86.2%, and the catalytic activity was 18,110 as calculated in terms of number of turnovers. The reaction product had an absolute configuration of (R) and an optical purity of 91.9%e.e.

### EXAMPLE 10

### Preparation of (R)-4-methyl-2-oxetanone:

The reaction procedure of Example 6 was followed except that 22.9 mg (0.0159 mmol) of the complex mixture catalyst obtained in Example 5 and 20.68 g (246.0 mmol) of 4-methylene-2-oxetanone were used. As a result, the desired compound was obtained in an amount of 13.5 g (yield: 65.2%). The percent conversion in this reaction was 65.2%, and the catalytic activity was 10,092 as calculated in terms of number of turnovers. The reaction product had an absolute configuration of (R) and an optical purity of 91.2%e.e.

### EXAMPLE 11

### Preparation of (R)-4-methyl-2-oxetanone:

The reaction procedure of Example 6 was followed except that 20.9 mg (0.0166 mmol) of the complex mixture catalyst obtained in Example 1 and 20.88 g (248.4 mmol) of 4-methylene-2-oxetanone were used under a hydrogen pressure of 2 MPa. As a result, the desired compound was obtained in an amount of 11.8 g (yield: 56.7%). The percent conversion in this reaction was 56.7%, and the catalytic activity was 8,508 as calculated in terms of number of turnovers. The reaction product had an absolute configuration of (R) and an optical purity of 93.0%e,e.

### EXAMPLE 12

### Preparation of (R)-4-methyl-2-oxetanone:

The reaction procedure of Example 7 was followed except that 18.9 mg (0.0120 mmol) of the complex mixture catalyst obtained in Example 6 and 21.1 g (252 mmol) of 4-methylene-2-oxetanone were used. As a result, the desired compound was obtained in an amount of 14.9 g (yield: 68.9%). The percent conversion in this reaction was 68.9%, and the catalytic activity was14,459 as calculated in terms of number of turnovers. The reaction product had an absolute configuration of (R) and an optical purity of 89.6%e.e.

### COMPARATIVE EXAMPLE 1

### Preparation of (R)-4-methyl-2-oxetanone:

A ruthenium-iodo-optically active phosphine complex described below and disclosed in JP-A-10-139791 was prepared. The ruthenium-iodo-optically active phosphine complex thus prepared was then used as a catalyst to prepare optically active 4-methyl-2-oxetanone. In some detail, into a 80 ml Schlenk's tube were charged 1.0 g (1.02 mmol) of [RuI₂(p-cymene)]₂, 1.41 g (2.07 mmol) of (S)-T-BINAP and 40 ml of methanol. The mixture was then stirred at a temperature of 55°C for 16 hours. After the termination of the reaction, methanol was then distilled off to obtain RuI₂[(S)-T-BINAP] in an amount of 2.35 g.

Into a reaction vessel were then charged 82.0 mg (0.0793 mmol) of RuI₂[(S)-T-BINAP], 21.3 g (253 mmol) of 4-methylene-2-oxetanone, 80 ml of tetrahydrofuran and 0.45 ml of deaerated water. The mixture was then allowed to undergo reaction at a hydrogen pressure of 4 MPa and a temperature of 60°C to obtain the desired compound in an amount of 13.8 g (yield: 65.0%). The percent conversion in this reaction was 65.0%, and the catalytic activity was 2,073 as calculated in terms of number of turnovers. The reaction product had an absolute configuration of (R) and an optical purity of 94.0%e.e.

### COMPARATIVE EXAMPLE 2

### Preparation of (R)-4-methyl-2-oxetanone:

Into a reaction vessel were charged 52.3 mg (0.0476 mmol) of a complex [RuI (p-cymene)((S)-SEGPHOS)]I prepared by a conventional method disclosed in JP-A-5-111639, 21.1 g (263 mmol) of 4-methylene-2-oxetanone, 80 ml of acetone and 0.45 ml of deaerated water. The mixture was then allowed to undergo reaction at a hydrogen pressure of 4 MPa and a temperature of 60°C to obtain the desired compound in an amount of 5.41 g (yield: 23.9%). The percent conversion in this reaction was 23.9%, and the catalytic activity was 1,318 as calculated in terms of number of turnovers. The reaction product had an absolute configuration of (R) and an optical purity of 91.8%e.e.

### COMPARATIVE EXAMPLE 3

### Preparation of (R)-4-methyl-2-oxetanone:

Into a reaction vessel were charged 52.4 mg (0.0476 mmol) of a complex [RuI((S)-BINAP) (CH₃CN)₃]⁺I⁻ prepared by a conventional method disclosed in *J*. *Chem. soc.,* Dalton Trans., pp. 2,099 - 2,107 (1992), 20.8 g (247 mmol) of 4-methylene-2-oxetanone, 80 ml of acetone and 0.45 ml of deaerated water. The mixture was then allowed to undergo reaction at a hydrogen pressure of 4 MPa and a temperature of 60°C to obtain the desired compound in an amount of 9.31 g (yield: 43.8%). The percent conversion in this reaction was 43.8%, and the catalytic activity was 2,278 as calculated in terms of number of turnovers. The reaction product had an absolute configuration of (R) and an optical purity of 93.5%e.e.

### COMPARATIVE EXAMPLE 4

Preparation of [Ru((S)-SEGPHOS) (CH₃CN)₄] (OhepDf)₂: The air in a 100 ml eggplant type flask was replaced by nitrogen. Into the eggplant type flask were then charged 12.0 mg (0.123 mmol) of [RuI₂(p-cymene)]₂, 15.2 mg (0.248 mmol) of (S)-SEGPHOS, 277 mg (0.515 mmol) of KOhepDf and 10 ml of deaerated acetonitrile. The mixture was then stirred at a temperature of 80°C for 16 hours. Thereafter, the reaction solution was cooled to a temperature of 40°C. Acetonitrile was then recovered under reduced pressure. In a nitrogen atmosphere, the residue was then stirred with 10 ml of methylene chloride and 15 ml of deaerated water for 10 minutes. The resulting methylene chloride layer was withdrawn through a syringe, and then washed with 15 ml of deaerated water. Methylene chloride was then distilled off under reduced pressure. The resulting complex was dried at a temperature of 30°C for 4 hours. As a result, the desired compound was obtained in an amount of 360 mg (yield: 78.3%).

³¹P NMR: 6 47.4

### Preparation of (R)-4-methyl-2-oxetanone:

The reaction procedure of Example 7 was followed except that 22.3 mg (0.119 mmol) of [Ru((S)-SEGPHOS) (CH₃CN)₄] (OhepDf)₂ obtained above and 21.6 g (256 mmol) of 4-methylene-2-oxetanone were used. As a result, the desired compound was obtained in an amount of 8.53 g (yield: 38.7%). The percent conversion in this reaction was 38.7%, and the catalytic activity was 8,332 as calculated in terms of number of turnovers. The reaction product had an absolute configuration of (R) and an optical purity of 85.1%e.e.

The catalytic activity (number of turnovers) of Examples 7 to 12 and Comparative Examples 1 to 4 and the absolute configuration and optical purity (%e.e.) of 4-methyl-2-oxetanone thus obtained are set forth in Table 1.

**Table 1**

| Example No. | Catalytic activity (number of turnovers) | Steric configuration | Optical purity (%e.e.) |
|---|---|---|---|
| Example 7 | 16,771 | R isomer | 91.6 |
| Example 8 | 11,594 | R isomer | 91.9 |
| Example 9 | 18,110 | R isomer | 91.9 |
| Example 10 | 10,092 | R isomer | 91.2 |
| Example 11 | 8,508 | R isomer | 93.0 |
| Example 12 | 14,459 | R isomer | 89.6 |
| Comparative Example 1 | 2,073 | R isomer | 94.0 |
| Comparative Example 2 | 1,318 | R isomer | 91.8 |
| Comparative Example 3 | 2,278 | R isomer | 93.5 |
| Comparative Example 4 | 8,332 | R isomer | 85.1 |

As can be seen in the foregoing results, the examples using the complex of the present invention ([RuIₐ(R¹-SEGPHOS)(sol)_{b}](I)_{c}(anion)_{d}) allow the reaction to proceed at a far higher rate than the comparative examples using conventionally known ruthenium-optically active phosphine complexes.

When used as a catalyst for asymmetric hydrogenation reaction, the novel ruthenium-iodo-optically active phosphine complex mixture obtained according to the present invention exhibits an extremely high catalytic activity and a high asymmetric yield in asymmetric reaction. In other words, the ruthenium-iodo-optically active phosphine complex mixture obtained according to the present invention can provide a product having a high optical purity. Thus, the ruthenium-iodo-optically active phosphine complex mixture obtained according to the present invention can be widely used as an economically favorable catalyst for asymmetric synthesis on an industrial basis. In particular, the asymmetric hydrogenation of 4-methylene-2-oxetanone in the presence of the complex mixture according to the present invention as a catalyst makes it possible to obtain optically active 4-methyl-2-oxetanone having a high optical purity useful as a starting material of polymer or the like at a high efficiency in a short period of time.

## Claims

1. A ruthenium-optically active phosphine complex mixture comprising three complexes represented by the following general formulae (I), (II) and (III), respectively:
[Ru(R¹-SEGPHOS) (sol)₄]I₂ (I)
[Ru(R¹-SEGPHOS) (sol)₄]I (anion) (II)
[RuI(R¹-SEGPHOS) (sol)₃] (anion) (III)
wherein sol represents a nitrile-based solvent; R¹-SEGPHOS represents an optically active tertiary phosphine represented by the following general formula (IV) : 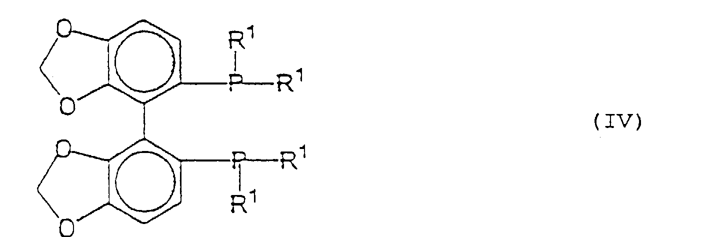 wherein R¹ represents an aryl group which may have a substituent selected from the group consisting of a C₁₋₄ lower alkyl group, a C₁₋₄ lower alkoxy group, a C₁₋₄ lower alkylamino group and a halogen atom, or a C₃₋₈ cycloalkyl group; and anion represents a perfluoroalkylsulfonyl anion.

2. A process for the preparation of a ruthenium-optically active phosphine complex mixture comprising three complexes represented by the following general formulae (I), (II) and (III), respectively:
[Ru(R¹-SEGPHOS) (sol)₄]I₂ (I)
[Ru(R¹-SEGPHOS) (sol)₄]I(anion) (II)
[RuI(R¹-SEGPHOS) (sol)₃] (anion) (III)
wherein sol represents a nitrile-based solvent; R¹-SEGPHOS represents an optically active tertiary phosphine represented by the following general formula (IV): 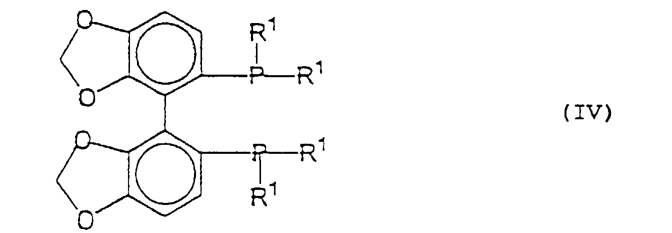 wherein R¹ represents an aryl group which may have a substituent selected from the group consisting of a C₁₋₄ lower alkyl group, a C₁₋₄ lower alkoxy group, a C₁₋₄ lower alkylamino group and a halogen atom, or a C₃₋₈ cycloalkyl group; and anion represents a perfluoroalkylsulfonyl anion,
which comprises reacting a ruthenium-optically active phosphine complex represented by the following general formula (V):
[RuI (arene) (R¹-SEGPHOS)]I (V)
wherein arene represents a hydrocarbon having a benzene ring; and R¹-SEGPHOS is as defined above,
with a perfluoroalkylsulfonic acid salt represented by the following general formula (VI)
Mₑ(anion)_{f} (VI)
wherein M represents a mono-, di- or trication selected from the group consisting of an alkaline metal cation, an alkaline earth metal cation and an ammonium cation; anion represents a perfluoroalkylsulfonyl anion; e is 1; and f is an integer of from 1 to 3, with the proviso that when M is a monocation, f is 1, when M is a dication, f is 2, and when M is a trication, f is 3, in a nitrile-based solvent.

3. A process for the preparation of a ruthenium-optically active phosphine complex mixture comprising three complexes represented by the following general formulae (I), (II) and (III), respectively:
[Ru(R¹-SEGPHOS)(sol)₄]I₂ (I)
[Ru(R¹-SEGPHOS) (sol)₄]I(anion) (II)
[RuI (R¹-SEGPHOS) (sol)₃] (anion) (III)
wherein sol represents a nitrile-based solvent; R¹-SEGPHOS represents an optically active tertiary phosphine represented by the following general formula (IV): 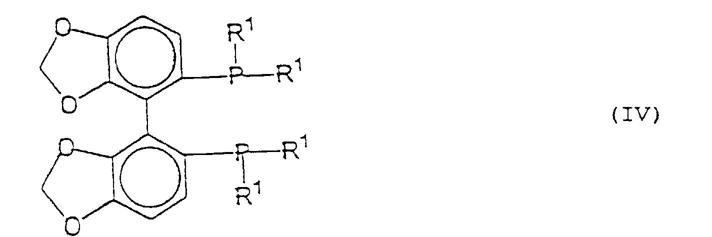 wherein R¹ represents an aryl group which may have a substituent selected from the group consisting of a C₁₋₄ lower alkyl group, a C₁₋₄ lower alkoxy group, a C₁₋₄ lower alkylamino group and a halogen atom, or a C₃₋₈ cycloalkyl group; and anion represents a perfluoroalkylsulfonyl anion ,
which comprises reacting a compound represented by the following general formula (VII):
[RuI₂(arene)]₂ (VII)
wherein arene represents a hydrocarbon having a benzene ring,
an optically active phosphine represented by the general formula (IV), and a perfluoroalkylsulfonic acid salt represented by the following general formula (VI):
Mₑ(anion)_{f} (VI)
wherein M represents a mono-, di- or trication selected from the group consisting of an alkaline metal cation, an alkaline earth metal cation and an ammonium cation; anion represents a perfluoroalkylsulfonyl anion; e is 1; and f is an integer of from 1 to 3, with the proviso that when M is a monocation, f is 1, when M is a dication, f is 2, and when M is a trication, f is 3,
in a nitrile-based solvent.

4. A catalyst for asymmetric hydrogenation of 4-methylene-2-oxetanone comprising as a main component a ruthenium-optically active phosphine complex mixture comprising three complexes represented by the following general formulae (I), (II) and (III), respectively:
[Ru(R¹-SEGPHOS) (sol)₄]I₂ (I)
[Ru(R¹-SEGPHOS)(sol)₄]I(anion) (II)
[RuI (R¹-SEGPHOS) (sol)₃] (anion) (III)
wherein sol represents a nitrile-based solvent; R¹-SEGPHOS represents an optically active tertiary phosphine represented by the following general formula (IV): 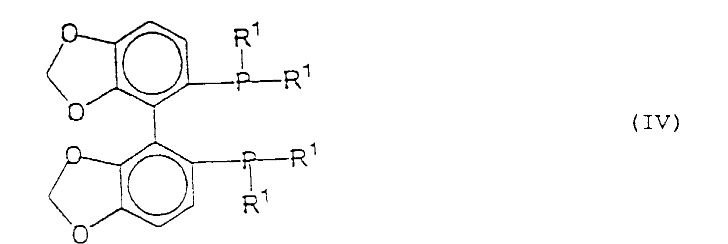 wherein R¹ represents an aryl group which may have a substituent selected from the group consisting of a C₁₋₄ lower alkyl group, a C₁₋₄ lower alkoxy group, a C₁₋₄ lower alkylamino group and a halogen atom, or a C₃₋₈ cycloalkyl group; and anion represents a perfluoroalkylsulfonyl anion.

5. A catalyst for asymmetric hydrogenation of 4-methylene-2-oxetanone which is a ruthenium-optically active phosphine complex mixture obtained by reacting a ruthenium-optically active phosphine complex represented by the following general formula (V):
[RuI (arene) (R¹-SEGPHOS)]I (V)
wherein arene represents a hydrocarbon having a benzene ring; and R¹-SEGPHOS represents an optically active tertiary phosphine represented by the following general formula (IV): 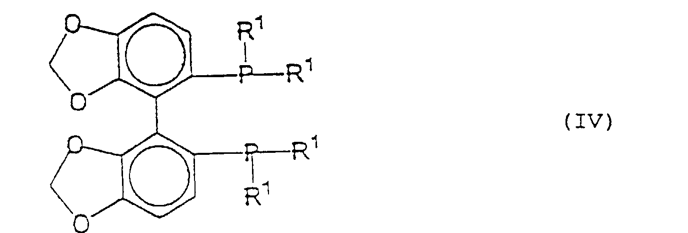 wherein R¹ represents an aryl group which may have a substituent selected from the group consisting of a C₁₋₄ lower alkyl group, a C₁₋₄ lower alkoxy group, a C₁₋₄ lower alkylamino group and a halogen atom, or a C₃₋₈ cycloalkyl group; and anion represents a perfluoroalkylsulfonyl anion,
with a perfluoroalkylsulfonic acid salt represented by the following general formula (VI)
Mₑ(anion)_{f} (VI)
wherein M represents a mono-, di- or trication selected from the group consisting of an alkaline metal cation, an alkaline earth metal cation and an ammonium cation; anion represents a perfluoroalkylsulfonyl anion; e is 1; and f is an integer of from 1 to 3, with the proviso that when M is a monocation, f is 1, when M is a dication, f is 2, and when M is a trication, f is 3,
in a nitrile-based solvent.

6. A catalyst for asymmetric hydrogenation of 4-methylene-2-oxetanone which is a ruthenium-optically active phosphine complex mixture obtained by reacting a compound represented by the following general formula (VII):
[RuI₂(arene)]₂ (VII)
wherein arene represents a hydrocarbon having a benzene ring,
an optically active phosphine represented by the following general formula (IV), 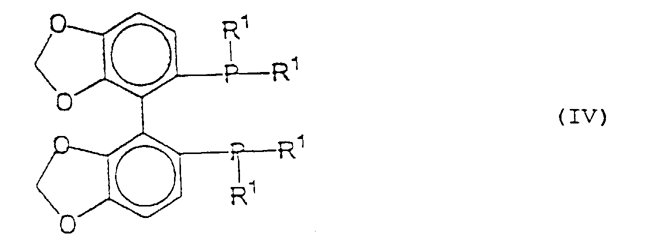 wherein R¹ represents an aryl group which may have a substituent selected from the group consisting of a C₁₋₄ lower alkyl group, a C₁₋₄ lower alkoxy group, a C₁₋₄ lower alkylamino group and a halogen atom, or a C₃₋₈ cycloalkyl group; and anion represents a perfluoroalkylsulfonyl anion,
and a perfluoroalkylsulfonic acid salt represented by the following general formula (VI):
Mₑ(anion)_{f} (VI)
wherein M represents a mono-, di- or trication selected from the group consisting of an alkaline metal cation, an alkaline earth metal cation and an ammonium cation; anion represents a perfluoroalkylsulfonyl anion; e is 1; and f is an integer of from 1 to 3, with the proviso that when M is a monocation, f is 1, when M is a dication, f is 2, and when M is a trication, f is 3,
in a nitrile-based solvent.
